# EUROPEAN PATENT SPECIFICATION

(11) **EP 4 465 965 B1**
(45) Date of publication and mention of the grant of the patent: **04.03.2026**
(21) Application number: 23701047.5
(22) Date of filing: 17.01.2023
(51) Int. Cl.: A61K 9/08, A61K 39/395, A61K 47/12, A61K 47/26, A61K 9/00

(54) **REDUCING POLYSORBATE DEGRADATION IN PROTEIN FORMULATIONS BY LOW AMOUNTS OF CITRIC ACID**
VERMINDERUNG DES POLYSORBATABBAUS IN PROTEINFORMULIERUNGEN DURCH GERINGE MENGEN VON ZITRONENSÄURE
RÉDUCTION DE LA DÉGRADATION DES POLYSORBATES DANS LES FORMULATIONS DE PROTÉINES PAR DE FAIBLES QUANTITÉS D'ACIDE CITRIQUE

(30) Priority: 19.01.2022 EP 22152311; 03.02.2022 EP 22155037; 24.10.2022 EP 22203349
(43) Date of publication of application: 27.11.2024
(60) Divisional application: 25192856.0
(73) Proprietor: Lonza Ltd, 3930 Visp (CH); Lonza Biologics plc, Slough Berkshire SL1 4DX (GB)
(72) Inventor: FEDOROWICZ, Filip, 4052 Basel (CH); HARTMANN, Lena, 4052 Basel (CH); KOULOV, Atanas, 4052 Basel (CH); SCHREINER, Viktoria, 4052 Basel (CH); LE BRUN, Virginie, 4052 Basel (CH); RAZVI, Abbas, 4052 Basel (CH); AUCAMP, Jean, Slough Berkshire SL1 4DX (GB); JAHN, Michael, 4052 Basel (CH)
(74) Representative: Greiner, Elisabeth
(86) International application number: PCT/EP2023/050944
(87) International publication number: WO 2023/139043

(56) References cited:
- US-A1- 2012 294 866
- GOPALRATHNAM G. ET AL.: "Impact of Stainless Steel Exposure on the Oxidation of Polysorbate 80 in Histidine Placebo and Active Monoclonal Antibody Formulation", vol. 72, no. 2, 23 March 2018 (2018-03-23), US, pages 163 - 175, XP055927796, ISSN: 1079-7440, Retrieved from the Internet <URL:http://dx.doi.org/10.5731/pdajpst.2017.008284> DOI: 10.5731/pdajpst.2017.008284
- HALL T. ET AL.: "Polysorbates 20 and 80 Degradation by Group XV Lysosomal Phospholipase A2 Isomer X1 in Monoclonal Antibody Formulations", JOURNAL OF PHARMACEUTICAL SCIENCES, vol. 105, no. 5, 1 May 2016 (2016-05-01), US, pages 1633 - 1642, XP055434435, ISSN: 0022-3549, DOI: 10.1016/j.xphs.2016.02.022
- LAM X.M. ET AL.: "Site-Specific Tryptophan Oxidation Induced by Autocatalytic Reaction of Polysorbate 20 in Protein Formulation", PHARMACEUTICAL RESEARCH, KLUWER ACADEMIC PUBLISHERS-PLENUM PUBLISHERS, NL, vol. 28, no. 10, 8 June 2011 (2011-06-08), pages 2543 - 2555, XP019950372, ISSN: 1573-904X, DOI: 10.1007/S11095-011-0482-X
- SHIRE S.: "Formulation of proteins and monoclonal antibodies mAbs", 1 January 2015, MONOCLONAL ANTIBODIES: MEETING THE CHALLENGES IN MANUFACTURING, FORMULATION, DELIVERY AND STABILITY OF FINAL DRUG PRODUCT,, PAGE(S) 93 - 120, XP009192951

## Description

### Technical Field

The present invention relates to a method for reducing polysorbate degradation by low amounts of citric acid in a protein formulation and a protein formulation with low amounts of citric acid having reduced polysorbate degradation.

### Technological Background

In the past 25 years antibodies have gained major significance in the pharmaceutical industry. Strickley et al., "A review of Formulations of Commercially Available Antibodies", J Pharm Sci. 2021; Jul;110(7):2590-2608, provides a review on 126 commercially available antibodies approved globally between 1986 and 2021, highlighting the importance of pharmaceutical protein formulations.

Polysorbates are surfactants which are added to pharmaceutical protein formulations in order to minimize interfacial stress, which might lead to protein aggregation or precipitation. Hence, polysorbates are critical stabilizers in pharmaceutical protein formulations. The most commonly polysorbates used in pharmaceutical protein formulations are polysorbate 20 (PS20) and polysorbate 80 (PS80), which consist of a core of dehydrated sorbitol, i.e. a mixture of sorbitan and isosorbide, that is polyethoxylated and esterified with fatty acids, mainly lauric acid in PS20 and oleic acid in PS80.

However, polysorbates such as PS20 and PS80 are prone to degradation in drug substances (DS) or drug products (DP) during storage. The degradation of polysorbates may have negative impacts on product quality in terms of (a) the occurrence of visible and/or sub-visible particulates due to insoluble matter of polysorbate degradants, (b) adverse impact on protein quality attributed to polysorbate degradation, such as peroxides leading to protein oxidation or induced protein aggregation by lauric acid, (c) reduced concentration of surfactant concentration, possibly leading to insufficient protection of the protein especially against interfacial stress, and (d) potential differences in safety considerations of the product e.g. due to degraded polysorbate.

Besides the isolation from natural sources, therapeutic proteins are mainly produced using mammalian or microbial host cell cultures, where the host cells express the target protein. In addition to the active protein, the host cells also produce host cell proteins (HCPs), which are in parts contained in the host cell culture supernatant, as described for example in Jahn, M. et al., PHARMACEUTICAL RESEARCH 2020, 37, 118, or in Champion, K., et al., BIOPROCESS INTERNATIONAL, 2005, 52-57. The downstream purification process typically includes various chromatographic and (ultra)filtration steps to capture and polish the therapeutic protein, and the purification process should be capable of depleting the majority of HCPs, at least to a degree where implications on safety for patients can be excluded. Yet, a 100% depletion of HCPs is, due to the natural principles of the separation and purification methods, which are based on equilibria, not possible. This implies that a certain amount of the HCPs is present in the bulk drug substance (DS), hence also in the DP.

It has been found that one root cause of polysorbate degradation is degradation catalyzed by lipases present in HCPs. For example, Hall et al., "Polysorbates 20 and 80 degradation by group XV lysosomal phospholipase A2 isomer X1 in monoclonal antibody formulations". J Pharm Sci. 2016;105:1633-42 reported that residual catalytic activity of lipases as part of HCPs contained in the final DP formulation can lead to polysorbate degradation by hydrolysis of the fatty acid ester linkages.

Therefore, residual lipolytic activity in DS and DP exerted by lipases present in HCPs due to sub-optimal depletion during the downstream purification process of the target protein can lead to hydrolysis of the polysorbate surfactants, leading to a compromised quality of the final therapeutic drug product.

The potential options for solving the problem polysorbate degradation due to residual lipolytic activity are 1) improvement of the downstream purification process in order to efficiently remove lipolytic enzymes, 2) generation of knock-out cell-lines for the expression of the active protein, where the cells lack expression of lipolytic enzymes, and 3) acceptance of a certain extent of polysorbate degradation and limiting the shelf life of the DP accordingly.

However, these potential options have several drawbacks in the sense that 1) additional efforts, time and costs for development work are needed, resulting in more complex, time consuming and more expensive downstream processes, 2) identification of the lipases responsible for polysorbate degradation in complex and time consuming proteomics studies are needed, requiring additional development work for generating knock-out cell lines and bringing about potentially reduced viability and efficiency of the resulting cells, and 3) skepticism of Health Authorities on the reduced product quality with potential rejections of submissions may be expected, or which may require the implementation of a complex supply chain for the DP having limited shelf life.

This warrants reducing polysorbate degradation in protein formulations without necessitating cost- and time-consuming measures or accepting a compromised protein formulation as explained above.

Hence, there is a need to provide a method that easily and effectively reduces polysorbate degradation in protein formulations and to provide a protein formulation having reduced polysorbate degradation.

The inventors surprisingly found that by using citric acid or a salt thereof in certain amounts, the polysorbate degradation in protein formulations can be reduced. Hence, the above needs are met by the subject matter of the present invention.

### Summary of the invention

In one aspect, the present invention provides a method for reducing polysorbate degradation in a protein formulation comprising a protein without lipolytic activity and a polysorbate, the method comprising adding citric acid or a salt thereof to the protein formulation at a concentration of from about 0.1 mM to about 5 mM, or from about 0.1 mM to about 0.9 mM.

In a further aspect, the present invention provides the use of citric acid or a salt thereof to reduce polysorbate degradation in a protein formulation comprising a protein without lipolytic activity and a polysorbate, wherein citric acid or a salt thereof is added to the protein formulation at a concentration of from about 0.1 mM to about 5 mM, or from about 0.1 mM to about 0.9 mM.

In a further aspect, the present invention provides a protein formulation having reduced polysorbate degradation, the protein formulation comprising a protein without lipolytic activity, a polysorbate, and citric acid or a salt thereof at a concentration of from about 0.1 mM to about 5 mM or of from about 0.1 mM to about 0.9 mM.

### Brief description of the figures

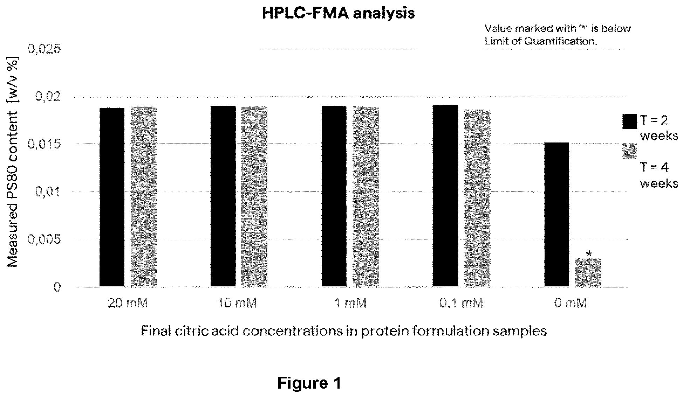

Figure 1: PS80 content in protein formulations with different citrate concentrations after incubation at 40°C for 2 weeks and 4 weeks.

### Detailed description

### Definitions

In order for the present invention to be readily understood, several definitions of terms used in the course of the invention are set forth below.

The term "protein" means a macromolecule composed of a sequence of amino acids. A protein can be a native protein, that is, a protein produced by a naturally-occurring and non-recombinant cell; or it can be produced by a genetically-engineered or recombinant cell, and comprise molecules having the amino acid sequence of the native protein, or molecules having deletions from, additions to, and/or substitutions of one or more amino acids of the native sequence, or molecules having the amino acid sequence of a protein with no relation to a native protein. The term also includes amino acid polymers in which one or more amino acids are chemical analogues of a corresponding naturally-occurring amino acid polymer. Proteins can include, e.g., antibodies, structural proteins, enzymes, membrane, membrane-associated, and/or transmembrane proteins, transporters, receptors, signaling proteins, and the like. Proteins also encompass modified proteins, e.g., conjugated to one or more non-peptide substances such as, e.g., a drug, a targeting moiety, a tag such as a visualization tag, and the like. A protein of the present invention can be a therapeutic protein, e.g., used in diagnosis, treatment, and/or prevention of a disease or disorder. In some embodiments, a polysorbate described herein can improve stability of the protein in a pharmaceutical formulation.

As used herein, the term "antibody" refers to a glycoprotein comprising at least two heavy (H) chains and two light (L) chains inter-connected by disulfide bonds, or an antibody fragment (antigen binding portion) thereof. Each heavy chain is comprised of a heavy chain variable region (VH) and a heavy chain constant region (CH). The heavy chain constant region is comprised of three domains, CH1, CH2 and CH3. Each light chain is comprised of a light chain variable region (VL) and a light chain constant region (CL). The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host cells or factors, including various cells of the immune system (e.g., effector cells) and the first component (C1q) of the classical complement system. Antibodies of the invention include monoclonal antibodies (including full length monoclonal antibodies) and polyclonal antibodies, whole antibodies, antibody-drug conjugates, chimeric antibodies, humanized antibodies, human antibodies or hybrid antibodies with dual or multiple antigen or epitope specificities, antibody fragments and antibody sub-fragments, e.g., Fab, Fab', F(ab')₂, fragments and the like, including hybrid fragments of any immunoglobulin or any natural, synthetic or genetically engineered protein that acts like an antibody by binding to a specific antigen to form a complex.

As used herein, the term "comprising" is to be construed as encompassing both "including" and "consisting of", both meanings being specifically intended, and hence individually disclosed, embodiments according to the present invention. "Comprising" in the sense of "including", likewise "having" or "containing", is inclusive or open-ended and does not exclude additional, unrecited elements or method steps.

As used herein, the articles "a" and "an" preceding an element or component are intended to be nonrestrictive regarding the number of instances (i.e. occurrences) of the element or component. Therefore, "a" or "an" is to be read to include one or at least one, and the singular word form of the element or component also includes the plural unless the number is obviously meant to be singular. For example, a protein formulation according to the present invention comprising a protein without lipolytic activity does not mean that said protein is necessarily the only protein comprised in the protein formulation, but it is to be understood that further proteins different to the protein without lipolytic activity may also be comprised in the protein formulation, e.g. proteins that have lipolytic activity. Accordingly, as used herein, the term "a protein without lipolytic activity" includes one or more "protein(s) without lipolytic activity", e.g. one, two, three, or four proteins without lipolytic activity; preferably one or two, more preferably one.

As used herein, the term "about" modifying the quantity of a substance, ingredient, component, or parameter employed refers to variation in the numerical quantity that can occur, for example, through typical measuring and handling procedures, e.g., liquid handling procedures used for making concentrates or solutions. Furthermore, variation can occur from inadvertent error in measuring procedures, differences in the manufacture, source, or purity of the ingredients employed to carry out the invention. In one embodiment, the term "about" means within 10% of the reported numerical value. In a more specific embodiment, the term "about" means within 5% of the reported numerical value.

The use of the term "or" in the claims is used to mean "and/or," unless explicitly indicated to refer only to alternatives or the alternatives are mutually exclusive, although the disclosure supports a definition that refers to only alternatives and "and/or."

The use of the term "for example" and its corresponding abbreviation "e.g." (whether italicized or not) means that the specific terms recited are representative examples and embodiments of the disclosure that are not intended to be limited to the specific examples referenced or cited unless explicitly stated otherwise.

In the context of the present invention, it is to be understood that "host cell proteins (HCPs)" does not refer to and include the "protein without lipolytic activity". Rather, "host cell proteins (HCPs)" are low-level, process-related undesired protein impurities derived from the host organism during manufacturing of the "protein without lipolytic activity", such as of a therapeutic protein. In the context of the present invention, the "protein without lipolytic activity" may also be referred to as "target protein"; the target protein being a protein without lipolytic activity. So the terms "target protein" and "protein without lipolytic activity" are used interchangeably herein; the target protein has no lipolytic activity.

It is to be understood that all embodiments, effects and findings described in the context of one particular aspect of the present invention, e.g. the inventive method, shall likewise apply to all other aspects of the present invention such as the inventive use and inventive protein formulation. For example, surprising findings described in the context of the inventive method likewise apply to the inventive use and inventive protein formulation.

As already outlined above, the present invention pertains to a method for reducing polysorbate degradation in a protein formulation comprising a protein without lipolytic activity and a polysorbate, the method comprising adding citric acid or a salt thereof to the protein formulation at a concentration of from about 0.1 mM to about 5 mM, or from about 0.1 mM to about 0.9 mM.

The inventors surprisingly found that by using citric acid or a salt thereof in certain amounts, the polysorbate degradation in protein formulations can be reduced. The method according to the present invention thus provides an excellent method for reducing polysorbate degradation in protein formulations. By using the inventive method, cumbersome root-cause investigations on polysorbate degradation can be avoided by directly reducing polysorbate degradation in a protein formulation without necessitating time-consuming and expensive development of cell lines or purification processes. Therefore, the method according to the present invention provides a convenient and efficient way to reduce polysorbate degradation in protein formulations.

The present invention further pertains to the use of citric acid or a salt thereof to reduce polysorbate degradation in a protein formulation comprising a protein without lipolytic activity and a polysorbate, wherein citric acid or a salt thereof is added to the protein formulation at a concentration of from about 0.1 mM to about 5 mM, or from about 0.1 mM to about 0.9 mM.

The present invention also pertains to a protein formulation having reduced polysorbate degradation, the protein formulation comprising a protein without lipolytic activity, a polysorbate, and citric acid or a salt thereof at a concentration of from about 0.1 mM to about 5 mM or of from about 0.1 mM to about 0.9 mM.

In one embodiment, the presence of citric acid in a protein formulation comprising a protein without lipolytic activity and a polysorbate reduces the degradation of polysorbate, when compared to a similar formulation without citric acid, by at least 5%, preferably by at least 10%, more preferably by at least 20%, after 2 weeks storage at 40 °C, and/or by at least 5%, preferably by at least 10%, more preferably by at least 25%, even more preferably by at least 50%, after 4 weeks storage at 40 °C.

Specifically, the inventors surprisingly found that already low concentrations of citric acid or a salt thereof effectively reduce polysorbate degradation in protein formulations.

According to the present invention, citric acid or a salt thereof is added to the protein formulation at a concentration of at least about 0.1 mM.

In one embodiment, citric acid or a salt thereof is added to the protein formulation at a concentration of up to about 5 mM, preferably up to about 4 mM, preferably up to about 3 mM, preferably up to about 2 mM, preferably up to about 1 mM, or up to about 0.9 mM.

Any of the lower limits can be combined with any of the upper limits.

Preferably, citric acid or a salt thereof is added to the protein formulation at a concentration of from about 0.1 mM to about 5 mM, e.g. at about 0.1 mM, at about 1 mM, or at about 5 mM. In one embodiment, citric acid is added to the protein formulation at a concentration of from about 0.1 mM to about 0.9 mM.

Hence, preferred concentrations of citric acid or a salt thereof in the protein formulation include from about 0.1 mM to about 5 mM, from about 0.1 mM to about 4 mM, from about 0.1 mM to about 3 mM, from about 0.1 mM to about 2 mM, from about 0.1 mM to about 1 mM, and from about 0.1 mM to about 0.9 mM.

It is to be understood that "citric acid or a salt thereof" for the purpose of the present invention refers to citric acid and salts of citric acid in their anhydrous forms as well as to their hydrate forms such as monohydrates, dihydrates, trihydrates, tetrahydrates, and pentahydrates. The term "citric acid or a salt thereof" is also to be understood to refer to only one type of citric acid or a salt thereof as well as to combinations of different types of citric acids (i.e. different hydrate forms) and/or different salts thereof. The term "citric acid or a salt thereof" is further to be understood to refer to citric acid, one or more salts of citric acids, and any mixtures thereof.

Suitable salts of citric acid include mono-, di- and tri-basic salts of citric acid and mixtures thereof, also optionally in combination with citric acid. Suitable cations for citric acid salts include sodium, potassium, calcium, magnesium, aluminium, N⁺R₁R₂R₃R₄; wherein
R₁, R₂, R₃ and R₄ are identical or different and independently from each other selected from the group consisting of H, C₁₋₄ alkyl, (CH₂)ₙOH;
n is 2, 3 or 4.

In one embodiment citric acid or a salt thereof is selected from the group consisting of citric acid, sodium citrates, potassium citrates, calcium citrates, and combinations thereof. Suitable sodium citrates include monosodium citrate, disodium citrate, trisodium citrate, and combinations thereof. Suitable potassium citrates include monopotassium citrate, dipotassium citrate, tripotassium citrate, and combinations thereof. Suitable calcium citrates include tricalcium dicitrate.

Preferably, citric acid or a salt thereof is selected from citric acid anhydrous, citric acid monohydrate, trisodium citrate dihydrate, and combinations thereof, more preferably from citric acid. Accordingly, in one embodiment, the present invention pertains to a method for reducing polysorbate degradation in a protein formulation comprising a protein without lipolytic activity and a polysorbate, the method comprising adding citric acid to the protein formulation at a concentration as defined herein.

In one preferred embodiment, the protein formulation is a liquid protein formulation. In such preferred embodiment, the method according to the present invention pertains to reducing polysorbate degradation in a liquid protein formulation by adding citric acid or a salt thereof to the protein formulation at a concentration as defined herein.

In one embodiment, the polysorbate comprises polysorbate-20, polysorbate-80, or a mixture thereof. In one embodiment, the polysorbate is polysorbate-20, polysorbate-80, or a mixture thereof. In one embodiment, the polysorbate is polysorbate-20. In one embodiment, the polysorbate is polysorbate-80. Accordingly, in one embodiment, reducing polysorbate degradation comprises reducing degradation of polysorbate-20 and/or polysorbate-80.

The amount of polysorbate in the protein formulation can be measured using a fluorescence micelle assay (FMA), such as a high-performance liquid chromatography fluorescence micelle assay (HPLC-FMA). Generally, every FMA coupled with HPLC suitable for detecting polysorbates such as PS20 and PS80, which is known in the art, can be used. HPLC-FMA can be carried out for example using a high-performance liquid chromatography (HPLC) system (Waters Alliance e2695) equipped with an isocratic pump, an auto sampler, a knitted reactor coil (1 mL; Supelco #57410-U) and a fluorescence detector (Waters 2475 FLR Detector).

Proteins, particularly therapeutic proteins, are mainly produced using mammalian or microbial cell cultures, where the cells express the target protein. In addition to the proteins, the cells also produce host cell proteins (HCPs), which are in parts contained in the cell culture supernatant. The downstream purification processes are typically not able to deplete 100% of the HCPs. This implies that a small percentage of the HCPs is present in the protein formulation. It is known that one root cause of polysorbate degradation is degradation catalyzed by lipases present in HCPs.

In one embodiment, polysorbate degradation may be caused by lipolytic activity. In one embodiment, lipolytic activity may be exerted by lipases. In one embodiment, the lipases may originate from host cell proteins (HCPs).

In one embodiment, the protein formulation comprises host cell proteins (HCPs). In one embodiment, lipolytic activity is exerted by host cell proteins (HCPs). In one embodiment, lipolytic activity is exerted by lipases which originate from host cell proteins (HCPs).

The lipolytic activity in the sense of the invention is a residual lipolytic activity which is not desired.

The amount of HCPs in the protein formulation can be determined by enzyme-linked immunosorbent assays (ELISA), as known to the skilled person, e.g. according to USP 39 NF 34 - General Chapter <1132> "Residual Host Cell Protein Measurement in Biopharmaceuticals".

In one embodiment, the protein formulation comprises HCPs as an impurity in trace amounts, for example 100 ppm or less of host cell proteins) based on the weight of the protein formulation. In one embodiment, the protein formulation comprises 95 ppm or less, such as 90 ppm or less, 85 ppm or less, 80 ppm or less, 75 ppm or less, 70 ppm or less, 65 ppm or less, or 60 ppm or less, of host cell proteins (HCPs) based on the weight of the protein formulation. In one embodiment, the protein formulation comprises 1-100 ppm of host cell proteins (HCPs) based on the weight of the protein formulation.

Preferably, the protein formulation comprises 100 ppm or less of host cell proteins (HCPs) based on the weight of the protein without lipolytic activity. If more than one protein without lipolytic activity is present in the protein formulation, it is to be understood that the amount of HCPs is based on the combined weight of proteins without lipolytic activity present in the protein formulation. In one embodiment, the protein formulation comprises 95 ppm or less, such as 90 ppm or less, 85 ppm or less, 80 ppm or less, 75 ppm or less, 70 ppm or less, 65 ppm or less, or 60 ppm or less, of host cell proteins (HCPs) based on the weight of the protein without lipolytic activity. In one embodiment, the protein formulation comprises 1-100 ppm of host cell proteins (HCPs) based on the weight of the protein without lipolytic activity.

Preferably, the protein formulation comprises 100 ppm or less of host cell proteins (HCPs) based on the weight of the target protein. If more than one target protein is present in the protein formulation, the amount of HCPs is based on the combined weight of target proteins present in the protein formulation. In one embodiment, the protein formulation comprises 95 ppm or less, such as 90 ppm or less, 85 ppm or less, 80 ppm or less, 75 ppm or less, 70 ppm or less, 65 ppm or less, or 60 ppm or less, of host cell proteins (HCPs) based on the total weight of the target protein. In one embodiment, the protein formulation comprises 1-100 ppm of host cell proteins (HCPs) based on the total weight of the target protein.

In one embodiment, the protein formulation comprises at least 1 µg/ml of the protein without lipolytic activity. In one embodiment, the protein formulation comprises at least 10 µg/ml of the protein without lipolytic activity. In one embodiment, the protein formulation comprises at least 100 µg/ml of the protein without lipolytic activity. In one embodiment, the protein formulation comprises at least 500 µg/ml of the protein without lipolytic activity. In one embodiment, the protein formulation comprises at least 1000 µg/ml of the protein without lipolytic activity.

In one embodiment, the protein formulation comprises at least 1 µg/ml of the target protein. In one embodiment, the protein formulation comprises at least 10 µg/ml of the target protein. In one embodiment, the protein formulation comprises at least 100 µg/ml of the target protein. In one embodiment, the protein formulation comprises at least 500 µg/ml of the target protein. In one embodiment, the protein formulation comprises at least 1000 µg/ml of the target protein.

The protein formulation preferably has as only source of lipolytic activity the HCPs.

According to the present invention, the protein formulation comprises a protein without lipolytic activity. Hence, said protein is not a lipase. Accordingly, the protein formulation comprises a protein without lipolytic activity and a polysorbate. Accordingly, the protein formulation comprises a protein that is not a lipase and a polysorbate. In one embodiment, the protein is a therapeutic protein. In such embodiment, the therapeutic protein does not have lipolytic activity, i.e. is not a lipase. Preferably, the therapeutic protein is an antibody. In one embodiment, the antibody is a monoclonal antibody. Preferably, the antibody is an anti-CD20 antibody. In one embodiment, the antibody is Rituximab.

Protein formulations are typically intended to be administered to a subject in need thereof. The route of administration of the inventive protein formulations is not particularly limited. In one embodiment, the protein formulation is administered to a subject in need thereof. In one embodiment, the protein formulation is for subcutaneous injection. In one embodiment, the protein formulation is a liquid protein formulation for subcutaneous injection. In one embodiment, the liquid protein formulation is administered as subcutaneous injection to a subject in need thereof.

Protein formulations that are usually buffered with citrate at high concentrations have the deficiency to cause injection site pain when administered as subcutaneous (sc) injection. The present invention circumvents this by using citric acid or a salt thereof at low concentrations (e.g. 0.1 mM - 20 mM), where likely no injection site pain will be observed after sc adminsitration, but still excellent reduction of polysorbate degradation is surprisingly achieved. Therefore, when the protein formulation is intended for subcutaneous injection, citric acid or a salt thereof is used in the present invention in low concentrations in the protein formulations to reduce polysorbate degradation, and optionally additional buffering agents for sc injections can be comprised in the protein formulation.

Thus, in one embodiment, the protein formulation further comprises a buffering agent, a salt, a stabilizer, or combinations thereof.

Suitable buffering agents include histidine, histidine hydrochloride, sodium succinate, and combinations thereof. Suitable stabilizers include trehalose, sucrose, mannitol, sorbitol, methionine, histidine, glycine, arginine, and combinations thereof.

The present invention will be further illustrated by the following examples, which should not be construed as limiting the present invention.

### Examples

In the following, the effect of various citrate concentrations on polysorbate degradation in a protein formulation were investigated.

### Materials

Citric acid monohydrate (ACS reagent, ≥99.0%), Sucrose (≥99.5%) and L-Histidine (L-His, ≥98.5%) were obtained from Sigma-Aldrich (now Merck KGaA). L-Histidine monohydrochloride monohydrate (L-His HCl, 99%) was obtained from AlfaAesar. Polysorbate 80 (PS80, NF, Multi-Compendial) was obtained from J.T. Baker.

A downstream purification sample of Rituximab Protein A: eluate with 18 g/L, was obtained as follows:
The cell culture material was obtained from a CHO SSI clone expressing Rituximab. The cell culture was clarified using a charged depth filter. The product was captured on a next-generation PrA resin. The Protein A eluate material was brought to pH 7.5, filtered over a 0.2 µm membrane, and stored at -20°C. The material was used without further purification.

### General procedure for preparation of protein formulation samples

A formulation buffer containing 25 mM L-His/L-His HCl at pH 6.0, 600 mM sucrose and 0.05 % (w/v) PS80 was prepared. The solution was filtered over a 0.22 µm PVDF Durapore^{®} sterile filter.

A second solution of 40 mM citric acid at pH 6.0 was prepared and filtered over a 0.22 µm PVDF Durapore^{®} sterile filter. In addition to the 40 mM citric acid stock solution, dilutions of the 40 mM citric acid solution at a target concentration of 20 mM, 2 mM and 0.2 mM using water were prepared.

Five protein formulation samples were prepared each by spiking 320 µL of the downstream purification sample (Rituximab Protein A eluate, 18 g/L) into 1680 µL of the above prepared formulation buffer in five different particle-free Nalgene bottles. Subsequently, 2000 µL of the respective citric acid solutions (40 mM, 20 mM, 2 mM and 0.2 mM) prepared above were added to four of the five bottles. The final protein formulations having five different citric acid concentrations (20 mM, 10 mM, 1 mM, 0.1 mM and 0 mM) were aliquoted into 2 mL HPLC-vials.

The final composition of the five protein formulation samples was 10.5 mM L-His/L-His HCl, 250 mM sucrose, 0.02 % (w/v) PS80, 1.4 mg/mL Rituximab Protein A eluate, at a pH of 6, with a final citric acid concentration 20 mM, 10 mM, 1 mM, 0.1 mM and 0 mM, respectively.

### Analyzing polysorbate degradation in protein formulation samples

1.2 mL of each of the protein formulation sample were aliquoted into particle-free Nalgene tubes and placed into a 40°C stability chamber. The samples were analysed after 2 week and 4 weeks of incubation using HPLC-FMA against a standard curve of intact PS80 in water.

The HPLC-FMA measurement was performed using a high-performance liquid chromatography (HPLC) system (Waters Alliance e2695) equipped with an isocratic pump, fluorescence detector (Waters 2475 FLR Detector), auto sampler and knitted reaction coil 1 mL (Supelco #57410-U). Samples were injected into a mobile phase containing 5 µM N-phenyl-1-napthylamine (NPN), 15 ppm (w/v) Brij^{®} 35 dissolved in a solution of 150 mM NaCl, 50 mM TRIS, 5% (v/v) acetonitrile at pH 8. Fluorescence was measured at λₑₓ = 350 nm and λₑₘ = 420 nm. Peak integration and analysis was carried out using the Empower 3 chromatographic data system (CDS).

The results of the protein formulations having five different citric acid concentrations (20 mM, 10 mM, 1 mM, 0.1 mM and 0 mM) after 2 weeks and 4 weeks storage at 40°C are shown in Figure 1.

As can be seen in Figure 1, in the protein formulation sample without citric acid (0 mM), PS80 concentration was significantly lower after 2 weeks and 4 weeks incubation at 40°C compared to the protein formulation samples comprising 0.1 mM, 1 mM, 10 mM, and 20 mM citric acid. This demonstrates that low amounts of citric acid are unexpectedly effective in reducing polysorbate degradation.

## Claims

1. A method for reducing polysorbate degradation in a protein formulation comprising a protein without lipolytic activity and a polysorbate, the method comprising adding citric acid or a salt thereof to the protein formulation at a concentration of from about 0.1 mM to about 5 mM, or from about 0.1 mM to about 0.9 mM.

2. The method according to claim 1, wherein the polysorbate comprises polysorbate-20, polysorbate-80, or a mixture thereof.

3. The method according to any one of claims 1 to 2, wherein the protein without lipolytic activity is a therapeutic protein.

4. The method according to any one of claims 1 to 3, wherein the protein formulation is for subcutaneous injection.

5. The use of citric acid or a salt thereof to reduce polysorbate degradation in a protein formulation comprising a protein without lipolytic activity and a polysorbate, wherein citric acid or a salt thereof is added to the protein formulation at a concentration of from about 0.1 mM to about 5 mM, or from about 0.1 mM to about 0.9 mM.

6. The use according to claim 5, wherein the polysorbate comprises polysorbate-20, polysorbate-80, or a mixture thereof.

7. The use according to claim 5 or 6, wherein the protein without lipolytic activity is a therapeutic protein.

8. The use according to any one of claims 5 to 7, wherein the protein formulation is for subcutaneous injection.

9. A protein formulation having reduced polysorbate degradation, the protein formulation comprising a protein without lipolytic activity, a polysorbate, and citric acid or a salt thereof at a concentration of from about 0.1 mM to about 5 mM, or from about 0.1 mM to about 0.9 mM.

10. The protein formulation according to claim 9, wherein the polysorbate comprises polysorbate-20, polysorbate-80, or a mixture thereof.

11. The protein formulation according to claim 9 or 10, wherein the protein without lipolytic activity is a therapeutic protein.

12. The protein formulation according to any one of claims 9 to 11, wherein the protein formulation is for subcutaneous injection.

13. The method, the use, or the protein formulation according to any one claims 1 to 12, wherein the protein formulation has as only source of lipolytic activity host cell proteins (HCPs).

14. The method, the use, or the protein formulation according to claim 13, wherein protein formulation comprises HCPs in an amount of 100 ppm or less based on the weight of the protein formulation.

15. The method, the use, or the protein formulation according to any one of claims 1 to 14, wherein the protein formulation comprises at least 1 µg/ml of the protein without lipolytic activity.

## Patentansprüche

1. Verfahren zur Verringerung des Polysorbatabbaus in einer Proteinformulierung, die ein Protein ohne lipolytische Aktivität und ein Polysorbat umfasst, wobei das Verfahren die Zugabe von Zitronensäure oder einem Salz davon zu der Proteinformulierung in einer Konzentration von etwa 0,1 mM bis etwa 5 mM oder von etwa 0,1 mM bis etwa 0,9 mM umfasst.

2. Verfahren nach Anspruch 1, wobei das Polysorbat Polysorbat-20, Polysorbat-80 oder eine Mischung davon umfasst.

3. Verfahren nach einem der Ansprüche 1 bis 2, wobei das Protein ohne lipolytische Aktivität ein therapeutisches Protein ist.

4. Verfahren nach einem der Ansprüche 1 bis 3, wobei die Proteinformulierung zur subkutanen Injektion bestimmt ist.

5. Verwendung von Zitronensäure oder einem Salz davon zur Verringerung des Polysorbatabbaus in einer Proteinformulierung, die ein Protein ohne lipolytische Aktivität und ein Polysorbat umfasst, wobei Zitronensäure oder ein Salz davon der Proteinformulierung in einer Konzentration von etwa 0,1 mM bis etwa 5 mM oder von etwa 0,1 mM bis etwa 0,9 mM zugegeben wird.

6. Verwendung nach Anspruch 5, wobei das Polysorbat Polysorbat-20, Polysorbat-80 oder eine Mischung davon umfasst.

7. Verwendung nach Anspruch 5 oder 6, wobei das Protein ohne lipolytische Aktivität ein therapeutisches Protein ist.

8. Verwendung nach einem der Ansprüche 5 bis 7, wobei die Proteinformulierung zur subkutanen Injektion bestimmt ist.

9. Proteinformulierung mit verringertem Polysorbatabbau, wobei die Proteinformulierung ein Protein ohne lipolytische Aktivität, ein Polysorbat und Zitronensäure oder ein Salz davon in einer Konzentration von etwa 0,1 mM bis etwa 5 mM oder von etwa 0,1 mM bis etwa 0,9 mM umfasst.

10. Proteinformulierung nach Anspruch 9, wobei das Polysorbat Polysorbat-20, Polysorbat-80 oder eine Mischung davon umfasst.

11. Proteinformulierung nach Anspruch 9 oder 10, wobei das Protein ohne lipolytische Aktivität ein therapeutisches Protein ist.

12. Proteinformulierung nach einem der Ansprüche 9 bis 11, wobei die Proteinformulierung zur subkutanen Injektion bestimmt ist.

13. Verfahren, Verwendung oder Proteinformulierung nach einem der Ansprüche 1 bis 12, wobei die Proteinformulierung als einzige Quelle lipolytischer Aktivität Wirtszellproteine (HCPs) aufweist.

14. Verfahren, Verwendung oder Proteinformulierung nach Anspruch 13, wobei die Proteinformulierung HCPs in einer Menge von 100 ppm oder weniger, bezogen auf das Gewicht der Proteinformulierung, umfasst.

15. Verfahren, Verwendung oder Proteinformulierung nach einem der Ansprüche 1 bis 14, wobei die Proteinformulierung mindestens 1 µg/ml des Proteins ohne lipolytische Aktivität umfasst.

## Revendications

1. Procédé permettant de réduire la dégradation du polysorbate dans une formulation de protéine comprenant une protéine sans activité lipolytique et un polysorbate, le procédé comprenant à ajouter de l'acide citrique ou un sel de celui-ci à la formulation de protéine à une concentration comprise entre environ 0,1 mM et environ 5 mM, ou entre environ 0,1 mM et environ 0,9 mM.

2. Procédé selon la revendication 1, dans lequel le polysorbate comprend du polysorbate-20, du polysorbate-80 ou un mélange de ceux-ci.

3. Procédé selon l'une quelconque des revendications 1 à 2, dans lequel la protéine sans activité lipolytique est une protéine thérapeutique.

4. Procédé selon l'une quelconque des revendications 1 à 3, dans lequel la formulation de protéine est destinée à une injection sous-cutanée.

5. Utilisation d'acide citrique ou d'un sel de celui-ci pour réduire la dégradation du polysorbate dans une formulation de protéine comprenant une protéine sans activité lipolytique et un polysorbate, dans laquelle l'acide citrique ou un sel de celui-ci est ajouté à la formulation de protéine à une concentration comprise entre environ 0,1 mM et environ 5 mM, ou entre environ 0,1 mM et environ 0,9 mM.

6. Utilisation selon la revendication 5, dans laquelle le polysorbate comprend du polysorbate-20, du polysorbate-80 ou un mélange de ceux-ci.

7. Utilisation selon la revendication 5 ou la revendication 6, dans laquelle la protéine sans activité lipolytique est une protéine thérapeutique.

8. Utilisation selon l'une quelconque des revendications 5 à 7, dans laquelle la formulation de protéine est destinée à une injection sous-cutanée.

9. Formulation de protéine présentant une dégradation réduite du polysorbate, la formulation de protéine comprenant une protéine sans activité lipolytique, un polysorbate et de l'acide citrique ou un sel de celui-ci à une concentration comprise entre environ 0,1 mM et environ 5 mM, ou entre environ 0,1 mM et environ 0,9 mM.

10. Formulation de protéine selon la revendication 9, dans laquelle le polysorbate comprend du polysorbate-20, du polysorbate-80 ou un mélange de ceux-ci.

11. Formulation de protéine selon la revendication 9 ou la revendication 10, dans laquelle la protéine sans activité lipolytique est une protéine thérapeutique.

12. Formulation de protéine selon l'une quelconque des revendications 9 à 11, dans laquelle la formulation de protéine est destinée à une injection sous-cutanée.

13. Procédé, utilisation ou formulation de protéine selon l'une quelconque des revendications 1 à 12, la formulation de protéine ayant comme seule source d'activité lipolytique des protéines de cellules hôtes (HCPs).

14. Procédé, utilisation ou formulation de protéine selon la revendication 13, la formulation de protéine comprenant des HCPs en une quantité inférieure ou égale à 100 ppm par rapport au poids de la formulation protéique.

15. Procédé, utilisation ou formulation de protéine selon l'une quelconque des revendications 1 à 14, la formulation de protéine comprenant au moins 1 pg/mL de la protéine sans activité lipolytique.
